(19) Europäisches Patentamt
European Patent Office
Office européen des brevets

(11) **EP 2 531 249 B1**

(12) **EUROPEAN PATENT SPECIFICATION**

(45) Date of publication and mention
of the grant of the patent:
**24.03.2021 Bulletin 2021/12**

(51) Int Cl.:
***A61M 16/10*** *(2006.01)*     ***A61M 16/12*** *(2006.01)*

(21) Application number: **11737816.6**

(22) Date of filing: **31.01.2011**

(86) International application number:
**PCT/US2011/023172**

(87) International publication number:
**WO 2011/094684 (04.08.2011 Gazette 2011/31)**

(54) **NITRIC OXIDE DELIVERY SYSTEM**

STICKOXIDAUSGABESYSTEM

SYSTÈME D'ADMINISTRATION D'OXYDE NITRIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **01.02.2010 US 300425 P**

(43) Date of publication of application:
**12.12.2012 Bulletin 2012/50**

(73) Proprietor: **VERO Biotech LLC
Cocoa, FL 32926 (US)**

(72) Inventors:
• **FINE, David, H.
Cocoa Beach, FL 32932 (US)**
• **DENTON, Ryan
Titusville, FL 32780 (US)**
• **VASQUEZ, Gregory
Cocoa, FL 32922 (US)**
• **JOHNSON, Bryan
Orlando, FL 32833 (US)**

(74) Representative: **D Young & Co LLP
120 Holborn
London EC1N 2DY (GB)**

(56) References cited:
**EP-A1- 2 501 427      WO-A1-97/36627
US-A1- 2006 180 147      US-A1- 2008 317 874
US-A1- 2009 314 289**

EP 2 531 249 B1

## Description

## TECHNICAL FIELD

This description relates to ambulatory and stationary methods and systems of using liquid nitrogen dioxide ($N_2O_4$) with a ventilator to generate and deliver nitric oxide to a patient.

## BACKGROUND

Nitric oxide (NO), also known as the nitrosyl radical, is a free radical that can be an important signalling molecule. For example, NO causes smooth muscles in blood vessels to relax, thereby resulting in vasodilation and increased blood flow through the blood vessel. These effects are limited to small biological regions since NO is highly reactive with a lifetime of a few seconds and is quickly metabolized in the body. Typically, NO gas can be supplied in a bottled gaseous form diluted in nitrogen gas ($N_2$). When delivered in this manner, great care has to be taken to prevent the presence of even trace amounts of oxygen ($O_2$) in the tank of NO gas because NO, in the presence of $O_2$, is oxidized into nitrogen dioxide ($NO_2$). Unlike NO, the part per million levels of $NO_2$ gas is highly toxic if inhaled and can form nitric and nitrous acid in the lungs.

EP 2 501 427 A1 citeable under Article 54(3) EPC discloses a device for delivery of a therapeutic amount of nitric oxide to an individual's lungs.

US 2008/317874 A1 discloses a nitric oxide delivery system which includes a gas bottle having nitrogen dioxide in air, converts nitrogen dioxide to nitric oxide and employs a surface- active material coated with an aqueous solution of antioxidant.

## SUMMARY

The present invention provides a system for delivering a therapeutic amount of nitric oxide to a patient as defined in the appended claims.

In one embodiment, a system for delivering a therapeutic amount of nitric oxide includes: ventilator for

delivering a gas mixture of oxygen and air; a liquid reservoir containing dinitrogen tetroxide; , a tube from the reservoir configured to connect to the gas supply being delivered to the patient, the tube having a bore size of about 25 microns or less; a first receptacle coupled to the tube, wherein the first receptacle includes a surface-activated material saturated with an aqueous solution of an antioxidant for converting nitrogen dioxide into nitric oxide; and a patient interface coupled to the first receptacle, wherein the first receptacle is configured to convert nitrogen dioxide into nitric oxide prior to reaching the patient interface; the system further comprising a gas mixer in communication with the reservoir and the gas mixture for ensuring proper mixing.

The reservoir can contain compressed nitrogen dioxide with or without a diluent gas. The receptacle can include a cartridge. The surface-activated material can be a silica gel, activated charcoal, activated carbon, activated alumina or calcium sulfate. The antioxidant can be a reducing agent, such as ascorbic acid, alpha tocopherol, or gamma tocopherol. The patient interface can be a mouth piece, face mask, or fully-sealed face mask or by means of tracheal intubation.

The system can further include a second receptacle wherein the second receptacle includes a surface-activated material saturated with an aqueous solution of an antioxidant. The system can further include a heating element associated with the reservoir. The system can further include a valve coupled to the reservoir and the tube. The tube can have a bore size of 10 microns or less.

Other features will become apparent from the following detailed description, taken in conjunction with the accompanying drawings, which illustrate by way of example, the features of the various embodiments.

## BRIEF DESCRIPTION OF THE DRAWINGS

FIG. 1 is a diagram of a NO delivery system.
FIG. 2 is a graph showing NO signal versus time during the course of a single breath from two cases.

## DETAILED DESCRIPTION

Nitric oxide (NO), also known as the nitrosyl radical, is a free radical that is an important signaling molecule in pulmonary vessels. Nitric oxide (NO) can moderate pulmonary hypertension caused by elevation of the pulmonary arterial pressure. Inhaling low concentrations of nitric oxide (NO), for example, in the range of 1-100 ppm can rapidly and safely decrease pulmonary hypertension in a mammal by vasodilation of pulmonary vessels.

Some disorders or physiological conditions can be mediated by inhalation of nitric oxide (NO). The use of low concentrations of inhaled nitric oxide (NO) can prevent, reverse, or limit the progression of disorders which can include, but are not limited to, acute pulmonary vasoconstriction, traumatic injury, aspiration or inhalation injury, fat embolism in the lung, acidosis, inflammation of the lung, adult respiratory distress syndrome, acute pulmonary edema, acute mountain sickness, post cardiac surgery acute pulmonary hypertension, persistent pulmonary hypertension of a newborn, perinatal aspiration syndrome, haline membrane disease, acute pulmonary thromboembolism, heparin-protamine reactions, sepsis, asthma and status asthmaticus or hypoxia. Nitric oxide (NO) can also be used to treat chronic pulmonary hypertension, bronchopulmonary dysplasia, chronic pulmonary thromboembolism and idiopathic or primary pulmonary hypertension or chronic hypoxia. NO can also be used to treat influenza. NO can further be used to inhibit the replication of the influenza virus in the lungs.

Generally, nitric oxide (NO) is inhaled or otherwise delivered to the individual's lungs. Providing a therapeutic dose of NO would treat a patient suffering from a disorder or physiological condition that can be mediated by inhalation of NO or supplement or minimize the need for traditional treatments in such disorders or physiological conditions.

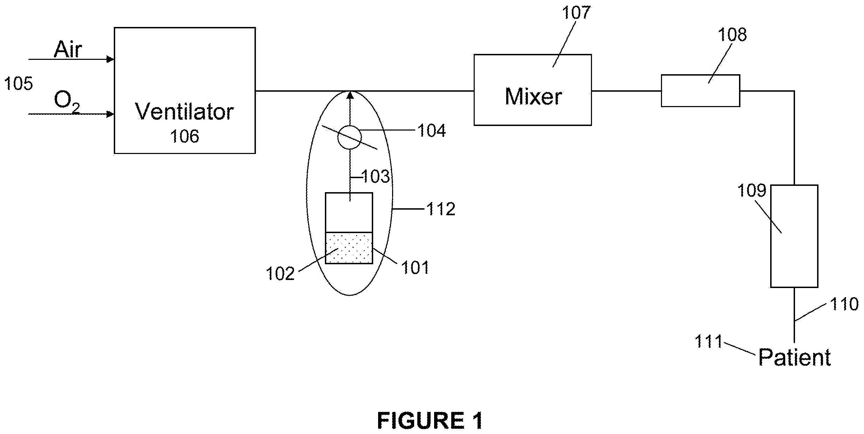

Currently, references on nitric oxide (NO) inhalation describe two solutions of solving the problem of fluctuating $NO_2$ concentration during NO delivery when the NO is supplied by means of a ventilator. First, the NO is diluted and mixed with oxygen upstream of the ventilator by means of a conventional blending valve. See for example, Figure 1 of Kirmse et al, Chest, Vol. 113, p. 1650-1657 (1998). The ventilator then pushes this premixed gas into the lungs. The result is that the NO is perfectly mixed and there are no concentration swings during the time of a typical breath, which may vary from approximately 1 to 4 seconds. The downside is that in the time that it takes for the gas mixture to pass through the ventilator and through the gas lines to the patient, considerable $NO_2$ can be formed (the rate of $NO_2$ formation is approximately 0.14 per second in 80 ppm NO and 90% oxygen). Since $NO_2$ is highly toxic, this approach generally leads to unacceptably high $NO_2$ levels. The NO, however is well mixed and there are no concentration gradients within the time frame (1 to 4 seconds) of a single breath (see for example, Kirmse et al., Chest, Vol. 113, p. 1650-1657 (1998), Schedin et al., British Journal of Anaesthesia, Vol. 82(2), p. 182-92 (1999), Imanaka et al., Anesthesiology, Vol. 86(3), p. 676-88 (1997), Nishimura et al., Anesthesiology, Vol. 82(5), p. 1246-54 (1995), Foubert et al., Anaesthesia, Vol. 54(3), p. 220-225 (1999)).

The second approach is one that is currently used, which is to inject the NO gas after the ventilator using computer control (see for example, U.S. Patent Nos. 5,373,693, 5,558,083, 5,732,694, 5,752,504, 6,089,229, 6,109,260, 6,125,846, 6,164,276, 6,581,592). The references describe measuring the instantaneous air flow rate with a hot wire anemometer or other very fast air flow measuring device, and sending this signal to the computer. The computer than calculates the timing on a valve which injects NO gas from a gas bottle containing NO in nitrogen (typically at 800 ppm) into the air/oxygen gas stream going to the patient. When the patient is exhaling, the valve is closed and when the patient is inhaling more and more NO can go into the circuit, so as to try and achieve a steady concentration of NO gas being delivered to the patient, during the breathing cycle.

As such, currently approved devices and methods for delivering inhaled NO gas require complex equipment and careful operation. The NO delivery system has to be purged to ensure that there is no $NO_2$ present in the delivery mechanism. NO gas is stored in heavy gas bottles with nitrogen and no traces of oxygen. Even then, the $NO_2$ impurity may be as high as 1% of the NO concentration and there is no way to remove this impurity and prevent it from reaching the patient along with the NO (at 80 ppm a 1% impurity could represent 0.8 ppm of $NO_2$ from this source). The NO gas is mixed with the air/oxygen gas mixture that is being delivered to the patient, by means of a specialized injector whose timing sequences are controlled by a microprocessor and which requires instantaneous measurement of the air/oxygen flow rate being delivered to the patient. All this equipment is required in order to minimize the oxidation of NO into nitrogen dioxide ($NO_2$) during the mixing and delivery process since $NO_2$ is highly toxic. There is also a need to monitor for $NO_2$ so as to ensure that the sensors and electronics are working properly. An oxygen analyzer is needed to ensure that the gas being delivered to the patient always has greater than 21% oxygen. A NO detector is also used to monitor the NO concentration. The equipment is also required to get the NO mixture to the lungs as soon as possible to minimize the formation of $NO_2$ in the delivery circuit. Even so, around 2 to 3 ppm (or higher) of $NO_2$ is produced at 80 ppm of NO and a high oxygen content. Typically, the injection point is where most of their $NO_2$ is formed, with NO at a high concentration, typically 800 ppm, going into a stream of air/oxygen. NO rich micelles mix with the air/oxygen and most $NO_2$ is formed at the micelle boundaries. The reason for this very rapid $NO_2$ formation is that the rate of reaction of NO with oxygen is proportional to the second power of NO and first power of Oxygen.

Because the NO has insufficient time to mix properly so as to minimize the formation of $NO_2$, concentration gradients of NO during the course of a breath result and are thought to be unavoidable. For example, during the course of a single pulse, at 20 liters per minute (average) flow, the flow rate to the lungs can vary from for example, 60 liters per minute down to zero. In some cases, the variation could be 120 liters per minute down to zero. If a steady flow of NO were to be introduced into this stream, instead of the introduction being computer controlled based on the instantaneous oxygen/air flow, then the concentration of NO would be expected to fluctuate widely during the course of a single breath, from many hundreds of ppm of NO down to zero, which would clearly be unacceptable.

The delivery devices disclosed herein use one of two sources of $NO_2$, a gas bottle containing $NO_2$ diluted in oxygen or air to approximately 800 ppm to 2000 ppm, or a liquid source containing pure $N_2O_4$ in which the $N_2O_4$ is vaporized to produce $NO_2$. In either case the $NO_2$ gas is introduced into the air/oxygen gas stream. Since $NO_2$ is being introduced, there is no concern about $NO_2$ formation. In one embodiment, the air/oxygen gas stream containing the $NO_2$ at the appropriate average concentration is then mixed and passed through a cartridge for generating NO by converting $NO_2$ to NO to generate NO gas. The NO containing air stream then passes though a second cartridge just prior to the inhalation by the patient, with the primary purpose of the second cartridge to remove any $NO_2$ that may have been formed in the gas lines after the first cartridge. A secondary use of the second cartridge is to provide 100% redundancy to the first cartridge in case of operational failure of the first cartridge. Both the gas bottle and liquid source platforms do not require sophisticated electronics, computers, measuring instantaneous air/oxygen flow going to the patient, a fast acting injecting valve, and monitoring equipment for $NO_2$ and oxygen. Additionally, the delivery devices are easy to use and do not require any specialized training. According to one embodiment, the NO delivery device uses a liquid $N_2O_4$ source which is the size of a coke can for one-time use or short-term treatments typically lasting from 1 to 24 hours or longer. Typical use involves reducing the NO concentration over time to zero as the patient is weaned off the NO gas. In one embodiment, the NO delivery device can deliver NO for 24 hours at 80 ppm NO at an average flow rate for example, of 15 L/min from a source of only 4 gram of liquid $N_2O_4$ (or less than 2.5 mL). In another embodiment, the NO delivery device is a gas bottle containing 1000 ppm of $NO_2$ in oxygen or air.

A system that includes the use of liquid nitrogen dioxide ($NO_2$), also called dinitrogen tetroxide ($N_2O_4$), as an $NO_2$ source is described. In one embodiment, the system works by storing liquid $N_2O_4$ in a reservoir which is heated. $N_2O_4$ boils at approximately 21°C. When the liquid reservoir is heated, the pressure rises. At 31°C the pressure in the reservoir is approximately 1 Atmosphere above ambient. Heating to 51°C raises the pressure in the reservoir to approximately 4 atmospheres. This is sufficient to drive the $NO_2$ vapor out of the reservoir and through a restriction (orifice) and into an air or oxygen stream. The concentration in the air or oxygen gas stream is dependent upon the flow rate of the air or oxygen stream, as well as on the temperature of the reservoir and the size of the orifice on the $N_2O_4$ reservoir. In another embodiment, a system described herein can include the use of a gas bottle containing $NO_2$ diluted in oxygen or air to approximately 800 ppm to 2000 ppm as an $NO_2$ source.

This device is ideal for generating a steady flow of $NO_2$ into the air stream, but has not been considered before as useful when connected to a ventilator that pulses air into the lungs at a typical frequency of 10 to 30 pulses (breaths) per minute.

As shown in FIG. 1, the liquid storage NO delivery system includes a heated reservoir 101. Generally, the reservoir 101 supplies NO lasting a few hours to one or more days of continuous use, depending upon the amount of liquid in the reservoir and the specific needs of the patient. In one embodiment, the reservoir 101 stores a therapeutic amount of $NO_2$ that is converted into NO by the cartridge 108. The therapeutic amount of NO is diluted to the necessary concentration. In various embodiments, the reservoir 101 is sized to hold tens of milligrams to tens of grams of liquid $N_2O_4$. For short-term treatments, the reservoir 101 can be sized to contain a few milligrams of $N_2O_4$. For example, the reservoir 101 may be sized to hold approximately 30 mg of $N_2O_4$ (101), which would provide 20 ppm of NO at 15 L/min for 60 minutes. For long-term applications, the reservoir 101 may be sized to contain 10 or more g of $N_2O_4$ for long-term use such as days to a week. For example, a reservoir containing approximately 7g of $N_2O_4$ may provide 20 ppm of NO at 20 L/min. for 7 days. In other examples, the reservoir 101 is sized to hold less then 1 ml, 2 ml, 3 ml, 4 ml, 5 ml, or 10 ml of liquid $N_2O_4$. In another embodiment, the liquid reservoir and its heated components 101-104 and 112 are replaced with a pressurized gas bottle containing $NO_2$ in air or oxygen. The flow out of the gas bottle can be controlled by a pressure regulator that is attached to the gas bottle, and/or by a fine control valve. The concentration of $NO_2$ in the gas bottle is typically in the range of 800 to 2000 ppm.

In one embodiment, the reservoir 101 can contain 1 g (about 0.7 ml) of $N_2O_4$ (102). The reservoir 101 can be attached to a tiny orifice or tube with a very narrow bore, 103. The reservoir 101 and the tube 103 can be covered by insulation. Since $N_2O_4$ boils at approximately 21°C, the pressure inside the reservoir would be approximately 15 psi at 30°C, 30 psi at 40°C and 60 psi at 50°C, for example. Instead of a gas regulator to control the pressure of the gas within a device, which is the conventional mechanism for use with a gas bottle, the temperature can be controlled such that the pressure inside the device is controlled precisely. In one embodiment, a heating element 112 can be associated with the reservoir and used to control the temperature. In another embodiment, a small microprocessor can be used to select the proper temperature such that the pressure inside the device and the release of $NO_2$ is controlled precisely. In one embodiment, the entire liquid system including elements 101-104 and 112 must be all be temperature controlled so that only $NO_2$ vapor is introduced into the air/oxygen flow.

As the gas vaporizes, one molecule of $N_2O_4$ forms two molecules of $NO_2$. Alternatively, using the known physical gas properties of $NO_2$, a critical orifice hole of about 3 to 4 microns would leak out $NO_2$ at about 0.16 ml per minute. If this 0.16ml of $NO_2$ were diluted into a gas stream of 2 liters per minute, the resulting concentration would be 80 ppm (parts per million). The same result can be achieved by using, for example, a quartz tube 103 with a 25 micron diameter bore size and about 20 inches long.

The pressure inside the reservoir 101 can be controlled very precisely by controlling the temperature. The flow rate Q out of the reservoir is proportional to the differential pressure, the fourth power of the diameter of the tube, and inversely proportional to the length of the tube. This equation was tested for this application:

$$Q = \frac{\Pi \Delta P D^4}{128\mu L}$$

In one embodiment for ambulatory use, a small ON/OFF valve 104 can be inserted between the reservoir and the fine control valve. In another embodiment, a check valve can be used such that the pressure on the spring of the check valve ensures that it remains tightly sealed at ambient temperatures. The valve can act as a variable sized hole or a simple needle valve. In one embodiment, a small microprocessor can be used to select the setting on the valve. In another embodiment, a quartz tube can be used to control the flow and all variations are controlled by varying the temperature only. This system would have no valve; resulting in an extremely simple device with just a reservoir which is heated to a known temperature and a fine tube. The device can be activated by heating the reservoir and cutting the tube to the desired length.

This device is designed to work with a ventilator in which an air/oxygen gas stream is pulsed into the lungs of a patient at a typical frequency of 15 to 30 pulses (breaths) per minute. The heated $N_2O_4$ source can leak a precisely controlled amount of $NO_2$ into the air/oxygen stream that leaves the ventilator. In one embodiment, the gasses are then passed through a gas mixer 107 to ensure proper mixing. The gasses then pass through a receptacle 108 which includes a surface-activated material saturated with an aqueous solution of an antioxidant. In one embodiment, the receptacle can be a cartridge for generating NO by converting $NO_2$ to NO. The cartridge, which may be referred to as a NO generation cartridge, includes an inlet and an outlet. For use with a ventilator the cartridges are designed to have a very low pressure drop of less than a few inches of water. Cartridges with this performance capability are manufactured by GeNO LLC. These cartridges contain a surface-active material that is soaked with a saturated solution of antioxidant in water to coat the surface-active material. The antioxidant can be a reducing agent, such as ascorbic acid, alpha tocopherol, or gamma tocopherol. The surface-active material can be silica gel.

In another embodiment, the NO gas can exit from the first receptacle into a second receptacle 109 which is typically identical to the former, except for the gas connections. The second receptacle can be an optional safety device used to ensure that all $NO_2$ is converted to NO before delivery to a patient. The function of the second receptacle is the same as the first receptacle and serves as a back up in case the first receptacle fails to convert $NO_2$ to NO. The mixture then flows directly to a patient interface 111. The patient interface can be a mouth piece, face mask, or fully-sealed face mask. The $NO_2$ concentration in the gas stream to the patient is always zero, even if the gas flow is delayed, since the second receptacle will convert any $NO_2$ present in the gas lines to NO.

[0028] In another embodiment, the NO gas can exit from the receptacle 107, into a NO sensor. The NO sensor can be an optional safety device used to assure that NO gas is flowing. The system illustrated in FIG. 1 can optionally include a $NO_2$ monitor, although this is not needed since the $NO_2$ concentration of the gas being delivered to the patient is typically zero.

In a further embodiment, the system of delivering NO gas can be used in a light, portable, ambulatory device for delivering NO with air. The device may be powered by a small, battery-driven pump or by patient inhalation (similar to smoking a cigar) and can be self-contained portable systems that do not require heavy gas bottles, sophisticated electronics, or monitoring equipment. Moreover, the delivery devices can allow an individual to self-administer a NO treatment. The delivery devices are also lightweight, compact, and portable. Alternatively, the NO delivery device can be a larger device, yet portable device that can deliver NO for longer periods of time. The ambulatory systems can include a mixing volume immediately after the $NO_2$ has been introduced into the air stream inside the ambulatory device itself so that the $NO_2$ can thoroughly mix.

Such ambulatory systems can further include a conserver to extend the useful life of the portable device. A conserver can sense the inhalation cycle and provide a gas flow of $NO_2$ gas but stop the flow during the exhalation process. Such conservers can double the lifetime of a portable NO gas bottle and provide a steady concentration of NO during the time frame of each breath. Conservers for portable systems are commercially available for example, from Pulmolab Medical Supplies and are known under trade names such as Bonsai OxyPneumatic Conserver, EasyPulse5 $O_2$ Conserving Regulator by Precision Medical, Lotus Electronic Oxygen Conserver with Alarm or without alarm, Oxymatic Electronic Every Breath 400 Series Conserver, Oxymatic Electronic Every Breath 400 Series Conserver Adjustable, Sage S.M.A.R.T. Therapy, Salter $O_2$ Express Pneumatic Conserver, Salter O2 Express Pneumatic Conserver with Safe-T Bag, Sequoia Electronic Alternate Breath Conserver, or Sequoia Electronic Every Breath Conserver. Other conservers can include Electronic Demand Pulsed-Dose delivery systems configured to deliver $NO_2$ to the patient by detecting the patient's inspiratory effort and providing gas flow during the initial portion of inspiration. As the patient initiates a breath, the cannula tip senses the flow, a solenoid valve opens, and a burst of oxygen is rapidly delivered to the patient. The size of the burst or flow can vary among different manufacturers. The pulsed-dose system can take the place of a flowmeter during NO therapy and can be attached to a gas source. In most devices the operator can select the gas flow and the mode of operation (either pulse or continuous flow). A battery-powered fluidic valve can be attached to a gaseous or liquid $NO_2$ supply to operate the system.

EXAMPLE: $NO_2$ was injected directly into the air/oxygen stream leaving the ventilator (Biomed (Crossvent 4+). The tidal volume was set to 1000 mL with 20 breaths per minute at a 1:2 I:E to achieve a 20 L/min flow with a peak flow of 60 L/min. The amount of $NO_2$ gas being delivered was controlled by controlling the temperature of the $N_2O_4$ reservoir and by means of a simple needle valve. The two GENO cartridges have considerable volume and also act to mix up the gas stream. A fast chemiluminescent detector was used to monitor the NO response. The NO detector was of our own design. The NO chemiluminescent detector was built from the parts of a TEA nitrosamine analyzer. It consisted of an ozone - sample reactor operating at 10 mm Hg pressure by means of a vacuum pump. The ozone and the sample were mixed in the ozone reactor in front of a cooled photomultiplier tube. The response of the photomultiplier tube was a measure of the NO concentration. The key to the high speed was to operate under vacuum and to have a fast amplifier that was taken from the TEA analyzer. The response time of the instrument was determined to be 10 milliseconds.

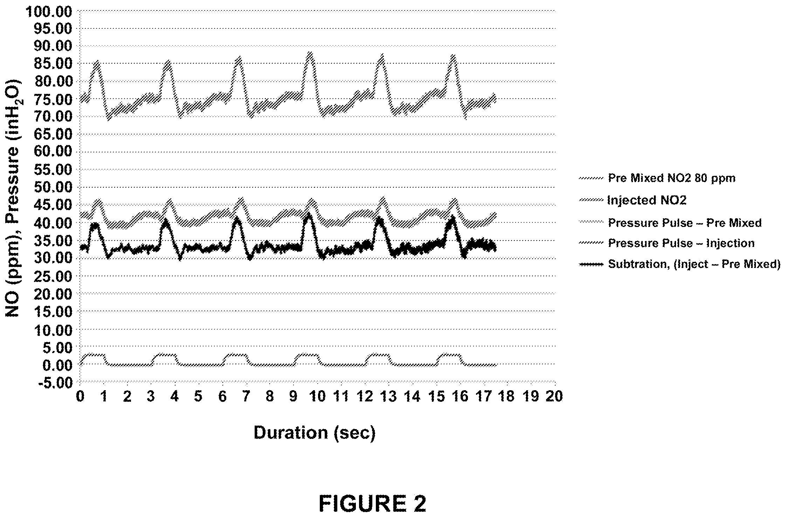

The perturbation in the NO signal versus time during the course of a single breath was compared for two cases (see Figure 2). The timing of the pressure pulses is shown in the bottom line, as measured by a pressure transducer. First, the response of the chemiluminescent NO analyser for the premixed condition of $NO_2$ in oxygen is shown as the second line from the top (blue). The pulses that are shown represent the effect of the pressure pulses of the ventilator on the chemiluminescent detector and do not reflect actual concentration gradients, since the gases were premixed. Second, the response of the chemiluminescent analyzer to $NO_2$ being introduced at a steady flow rate down stream of the ventilator and after the two ascorbic acid cartridges is shown in the line at the top of the page (red). When the raw data for the two lines are subtracted from each other, the difference is shown as the third line from the top (black). All of the data was collected at 80 ppm, the offsets are shown in the figure are for clarity only. The subtracted line has a peak to peak noise of 10 ppm of NO. The difference from the mean is + 7 ppm (+8.8%) and -3 ppm (-3.8%), which means that at 80 ppm the peak was 87 and the low 77 ppm. This is far superior to what the FDA guidance document deems acceptable which was a swing of +150% of the average NO level down to zero. When the response time of the instrument was electronically slowed down to 170 milliseconds, the premixed and the post ventilator lines showed no perturbation. When using an electrochemical detector, PrinterNOx, with a time constant of approximately 30 seconds, no differences were observed.

The system was used in preliminary animal studies using pigs. The anesthetised animal was placed was placed on a Crossvent 4+ ventilator (Bio-Med Devices) connected to the NO delivery system described here. With this ventilator, the air and oxygen was mixed prior to the ventilator by means of a medical air - oxygen gas blender. The pig was stabilized for 30 minutes prior to induction of pulmonary hypoxemia that was induced by decreasing the inspired concentration of oxygen to 15% from a normoxic level of 30%. Hypoxia was maintained for approximately 10 to 15 minutes prior to treatment with specific doses of inhaled NO therapy. The NO was delivered by the GeNO system described herein at either 1, 5, 20 or 80 PPM for 10 to 15 minutes. The desired NO dose was delivered by turning on the flow of $NO_2$ and adjusting the air /oxygen blender so that the ventilator delivered the precise $NO_2$ concentration to the ascorbic acid cartridge for generation of NO, just prior to inhalation. The inspired concentration of NO and $NO_2$ was continuously monitored by removing a 250ml/min in a side stream to an electrochemical gas analyzer (PrinterNox, Micro Medical Limited, Kent, UK). Following each treatment with NO, the $FIO_2$ was returned to 30% for at least 30 minutes.

The effects of inhaled NO produced by the GeNO ascorbic acid cartridge on mean pulmonary artery pressure (mPAP) induced by hypoxemia in swine showed that a reduction of inspired oxygen from 30% to 15% increased mPAP by approximately 40%, whereas inhaled NO from the GeNO system significantly reduced the elevated mPAP induced by hypoxia. Reduction of inspired oxygen from 30 to 15% increased PVR by approximately 30%, whereas the delivery of inhaled NO via the GeNO system reduced this hypoxemia induced PVR elevation to near baseline conditions. Systemic vascular resistance (SVR) and mean arterial pressure (MAP) were not significantly affected by the induction of hypoxemia nor the delivery of inhaled NO. Throughout these in vivo experiments, there was no significant delivery of $NO_2$. Even at 80 ppm, the $NO_2$ level was < 0.05ppm, which is the detection limit of the instrument.

The various embodiments described above are provided by way of illustration only and should not be construed to limit the claimed invention. Those skilled in the art will readily recognize various modifications and changes that may be made to the claimed invention without following the example embodiments and applications illustrated and described herein, and without departing from the scope of the claimed invention, which is set forth in the following claims.

## Claims

1. A system for delivering a therapeutic amount of nitric oxide to a patient, comprising:

   a ventilator (106) for delivering a gas mixture of oxygen and air;
   a liquid reservoir (101) containing dinitrogen tetroxide (102);

a tube (103) from the reservoir (101) configured to connect to the gas supply being delivered to the patient, the tube (103) having a bore size of about 25 microns or less;

a first receptacle (108) coupled to the tube (103), wherein the first receptacle (108) comprises a surface-activated material saturated with an aqueous solution of an antioxidant for converting nitrogen dioxide into nitric oxide; and

a patient interface (111) coupled to the first receptacle (108), wherein the first receptacle (108) is configured to convert nitrogen dioxide into nitric oxide prior to reaching the patient interface (111); the system further comprising a gas mixer (107) in communication with the reservoir (101) and the gas mixture for ensuring proper mixing.

2. The system of claim 1, wherein the reservoir (101) contains compressed nitrogen dioxide with or without a diluent gas.

3. The system of claim 1 or 2, further comprising a heating element (112) associated with the reservoir (101).

4. The system of any one of claims 1-3, further comprising a valve (104) coupled to the reservoir (101) and the tube (103).

5. The system of any one of claims 1-4, wherein the tube (103) has a bore size of 10 microns or less.

**Patentansprüche**

1. System zur Abgabe einer therapeutischen Menge an Stickstoffmonoxid an einen Patienten, umfassend:

ein Beatmungsgerät (106) zur Abgabe einer Gasmischung von Sauerstoff und Luft,

ein Flüssigkeitsreservoir (101), das Distickstofftetroxid (102) enthält,

einen Schlauch (103) vom Reservoir (101), der so konfiguriert ist, dass er mit der an den Patienten abgegebenen Gaszufuhr verbunden ist, wobei der Schlauch (103) eine Bohrweite von etwa 25 Mikron oder weniger aufweist,

ein erstes Aufnahmegefäß (108), gekuppelt mit dem Schlauch (103), wobei das erste Aufnahmegefäß (108) ein mit einer wässrigen Lösung eines Antioxidationsmittels zur Umwandlung von Stickstoffdioxid in Stickstoffmonoxid gesättigtes oberflächenaktives Material umfasst, und

ein Patienten-Interface (111), gekuppelt mit dem ersten Aufnahmegefäß (108), wobei das erste Aufnahmegefäß (108) so konfiguriert ist,

dass es Stickstoffdioxid vor dem Erreichen des Patienten-Interface (111) in Stickstoffmonoxid umwandelt,

wobei das System weiterhin einen Gasmischer (107) in Kommunikation mit dem Reservoir (101) und der Gasmischung zur Sicherstellung einer richtigen Durchmischung umfasst.

2. System nach Anspruch 1, wobei das Reservoir (101) komprimiertes Stickstoffdioxid mit oder ohne Verdünnungsgas enthält.

3. System nach Anspruch 1 oder 2, weiterhin umfassend ein mit dem Reservoir (101) assoziiertes Heizelement (112) .

4. System nach einem der Ansprüche 1-3, weiterhin umfassend ein mit dem Reservoir (101) und dem Schlauch (103) gekuppeltes Ventil (104).

5. System nach einem der Ansprüche 1-4, wobei der Schlauch (103) eine Bohrweite von 10 Mikron oder weniger aufweist.

**Revendications**

1. Système d'administration d'une quantité thérapeutique d'oxyde nitrique à un patient, comprenant :

un ventilateur (106) pour administrer un mélange gazeux d'oxygène et d'air ;

un réservoir de liquide (101) contenant du tétraoxyde de diazote (102) ;

un tube (103) partant du réservoir (101), configuré pour se raccorder à l'alimentation en gaz administrée au patient, le tube (103) ayant une taille d'alésage d'environ 25 microns ou moins ;

un premier récipient (108) accouplé au tube (103), le premier récipient (108) comprenant un matériau à surface activée saturé avec une solution aqueuse d'un antioxydant pour convertir le dioxyde d'azote en oxyde nitrique ; et

une interface avec un patient (111) accouplée au premier récipient (108),

le premier récipient (108) étant configuré pour convertir le dioxyde d'azote en oxyde nitrique avant qu'il n'atteigne l'interface avec le patient (111) ;

le système comprenant en outre un mélangeur de gaz (107) en communication avec le réservoir (101) et avec le mélange de gaz pour assurer un bon mélange.

2. Système selon la revendication 1, dans lequel le réservoir (101) contient du dioxyde d'azote comprimé avec ou sans gaz diluant.

3. Système selon la revendication 1 ou 2, comprenant en outre un élément chauffant (112) associé au réservoir (101).

4. Système selon l'une quelconque des revendications 1 à 3, comprenant en outre une soupape (104) accouplée au réservoir (101) et au tube (103).

5. Système selon l'une quelconque des revendications 1 à 4, dans lequel le tube (103) présente une taille d'alésage de 10 microns ou moins.

FIGURE 1

FIGURE 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2501427 A1
- US 2008317874 A1
- US 5373693 A
- US 5558083 A
- US 5732694 A
- US 5752504 A
- US 6089229 A
- US 6109260 A
- US 6125846 A
- US 6164276 A
- US 6581592 B

### Non-patent literature cited in the description

- **KIRMSE et al.** *Chest,* 1998, vol. 113, 1650-1657
- **SCHEDIN et al.** *British Journal of Anaesthesia,* 1999, vol. 82 (2), 182-92
- **IMANAKA et al.** *Anesthesiology,* 1997, vol. 86 (3), 676-88
- **NISHIMURA et al.** *Anesthesiology,* 1995, vol. 82 (5), 1246-54
- **FOUBERT et al.** *Anaesthesia,* 1999, vol. 54 (3), 220-225